# EUROPEAN PATENT APPLICATION

(11) **EP 2 332 637 A1**
(43) Date of publication of application: **15.06.2011**
(21) Application number: 09014778.6
(22) Date of filing: 27.11.2009
(51) Int. Cl.: B01D 69/02, B01D 69/08, B01D 67/00, B29C 59/14, A61L 33/00

(54) **Hollow fibers with selective, plasma induced modification of lumen surface and continuous process for their production**

(71) Applicant: Gambro Lundia AB, 220 10 Lund (SE); Fraunhofer-Gesellschaft zur Förderung der angewandten Forschung, 80686 München (DE)
(72) Inventor: Rempfer, Martin, 72810 Gomaringen (DE); Storr, Markus, 70794 Filderstadt (DE); Winz, Cornelia, 72108 Rottenburg-Weiler (DE); Klotz, Manuela, 72379 Hechingen (DE); Freudemann, Wolfgang, 72379 Hechingen (DE); Barz, Jakob, 70199 Stuttgart (DE); Elkin, Ben, 70794 Filderstadt (DE); Müller, Michael, 70599 Stuttgart (DE); Oehr, Christian, 71083 Herrenberg (DE); Panowitz, Sarah, 72660 Beuren (DE)
(74) Representative: Hössle Patentanwälte Partnerschaft

(57) **Abstract**

The present disclosure relates to a continuous process for selectively modifying the lumen surface of a hollow fiber membrane using a plasma, an apparatus for carrying out the process and to hollow fiber membranes obtainable by the process and their use.

## Description

### Technical Field

The present disclosure relates to a continuous process for selectively modifying the lumen surface of a hollow fiber membrane using a plasma-chemical discharge, an apparatus for carrying out the process and to hollow fiber membranes obtainable by the process and their use.

### Description of the Related Art

Hollow fiber membranes are frequently comprised of relatively inert polymeric materials which require the use of aggressive chemical agents for modification of their surface by conventional wet chemistry. The chemical composition of the membrane is substantially homogeneous and results in similar properties of the inner and outer surfaces. Wet-chemical processes require a good wettability of the surface to be modified. The reaction medium is spread over the complete surface, the effect being enhanced by the capillary action of the pores. Therefore, usually the surface of a porous hollow fiber membrane is homogeneously modified by such a process.

A plasma-chemical discharge, on the other hand, allows for a localized coating or functionalization of porous membranes, as reactive species only form where the discharge is generated. This is true both for flat sheet membranes and for hollow fiber membranes. While a localized plasma treatment of flat sheet membranes is easily achieved, the selective modification of the lumen surface of a hollow fiber is difficult, in particular when the inner diameter of the fiber is less than 2 mm.

EP 0 622 111 A1 discloses a batch process for the plasma-induced surface modification of the surface on the inside of hollow substrates having a very small diameter (≤ 2 mm). *Inter alia,* the modification of the lumen surface of microporous hollow fibers made of polysulfone or polyethersulfone is disclosed. In the working examples, polymer fibers having an inner diameter of from 0.6 to 1.2 mm are modified. In Example 14, the lumen surface of polyethersulfone hollow fibers coated with a polysiloxane-polyamide copolymer is modified with an argon plasma. The specification mentions helium and other noble gases, ammonia, nitrogen, oxygen, water, carbon monoxide, nitrogen oxides and sulfur dioxide as examples of non-polymerizable gases suitable for plasma production.

EP 0 622 111 A1 teaches a process which uses a tubular reactor having an inner diameter which is only slightly larger than the outer diameter of the hollow fiber to be treated. A reactive gas is introduced into the hollow fiber at a rate of from 0.1 to 50 sccm/min (preferably 1 to 10 sccm/min), at a pressure of 200 to 5.000 mTorr [0.3 to 6.7 mbar], (preferably 600 to 1.900 mTorr [0.8 to 2.5 mbar]), and a plasma is ignited and maintained inside the fiber by a RF discharge having from 10 to 500 W (preferably 50 to 150 W). The radio frequencies employed range from 0,1 to 100 MHz, 13.56 MHz being preferred.

WO 2006/006918 discloses a continuous process for the re-gio-selective plasma-induced modification of the outer surface on the filtrate side and the pores of a porous hollow fiber membrane. The surface of the lumen of the hollow fiber remains unaffected, as no plasma is ignited inside the hollow fiber (particularly because the cross section dimension of the plasma volume circumferential to the outer wall of the hollow fiber is significantly larger than the diameter of the lumen). The reactive gas flows into the lumen of the hollow fiber from the outside through the pores. The inner diameter of the hollow fibers ranges from 0.07 to 0.7 mm, preferably 0.2 to 0.4 mm, the outer diameter is 0.11 to 1 mm, preferably 0.3 to 0.8 mm, in particular 0.42 mm. Among others, hollow fibers comprising a mixture of polyarylethersulfone and polyvinylpyrrolidone are mentioned.

The process taught by WO 2006/006918 uses a tubular reactor having several compartments in which gas pressure is reduced and increased again in steps. The compartments are separated by aperture plates having an inner diameter of from 0.05 to 0.8 mm, which is preferably 0.2 mm larger than the outer diameter of the hollow fiber. The gas pressure in the central compartment, in which the plasma is ignited, ranges from 0.05 to 10 mbar, and is preferably 0.25 mbar, after the reactive gas has been introduced. WO 2006/006918, Fig. 2 discloses a pressure distribution, wherein the pressure in the central compartment filled with reactive gas is higher than the pressure in the adjacent compartments. The reactive gas may comprise amino, carboxyl, aldehyde, ester, epoxy or sulfonic acid functionalities. Ammonia is mentioned as a suitable gas. The reactive gas may be mixed with a carrier gas like helium, nitrogen, hydrogen or argon. The radio frequencies used for igniting and maintaining the plasma range from 1 kHz to 13,56 MHz or multiples thereof, 13.56 MHz being preferred. Power input is 0.5 to 50 W, preferably 20 W. The hollow fiber is transported through the reactor at a speed of 0.1 to 100 m/min, preferably 10 m/min. Residence time in the central compartment is 0.5 to 30 s.

### Summary

It is an object of the present invention to provide a continuous process for selectively modifying the surface of the lumen of a hollow fiber membrane.

The process of the invention involves selective chemical modification of the lumen surface of a porous hollow fiber membrane in a low-temperature plasma by continuous air-to-air treatment.

It is also an object of the present invention to provide hollow fiber membranes having specific functional groups on their lumen surface which are not present on the other surfaces of the hollow fiber membranes.

It has been found that such fibers can be obtained by selectively modifying the lumen surface of a hollow fiber membrane using a low-temperature plasma.

It is also an object of the present invention to provide an apparatus for carrying out the process of the present invention.

The present invention also relates to a porous hollow fiber membrane produced by the process of the present invention, an adsorption device containing such a porous hollow fiber membrane, and the use of such a porous hollow fiber membrane.

### Brief Description of the Drawings

Figure 1 shows a schematic view of an embodiment of the apparatus of the invention in use.
Figure 2 shows a schematic cross-sectional view of the plasma chamber of an embodiment of the apparatus of the invention.
Figure 3 shows a schematic cross-sectional view of an embodiment of a fiber transport mechanism for use in the apparatus of the invention.
Figure 4 shows a schematic view of another embodiment of a fiber transport mechanism for use in the invention.

### Detailed Description

One embodiment of the process of the invention uses a modification of the apparatus disclosed in WO 2006/006918, incorporated herein by reference. The porous hollow fiber membrane is passed through a set of consecutive pressure compartments with a view to setting the ambient pressure from atmospheric pressure to a vacuum in line and after the plasma ignition in a corresponding way increasing the ambient pressure back to an atmospheric pressure.

Alternatively, a set of consecutive compartments filled with a plasma precursor gas composition can be used, wherein the atmospheric gas is gradually replaced by the plasma precursor gas composition.

In one embodiment of the process of the invention, a hollow fiber membrane is continuously transported through a zone of reduced pressure having a negative pressure gradient, or through a multitude of zones of stepwise reduced pressure, thereby removing gas from the hollow fiber membrane. The hollow fiber membrane then is continuously transported through a plasma ignition zone comprising a reactive gas, optionally admixed with a carrier gas, and a plasma is generated inside the lumen of the hollow fiber membrane within the plasma ignition zone, thereby generating functional groups exclusively on the lumen surface of the hollow fiber membrane. The hollow fiber membrane is then continuously transported through a zone of reduced pressure having a positive pressure gradient.

The process uses a tubular reactor having several compartments in which gas pressure is reduced and increased again in steps. The compartments are separated by aperture plates having an inner diameter of from 0.05 to 0.8 mm, e.g., 0.4 to 0.7 mm.

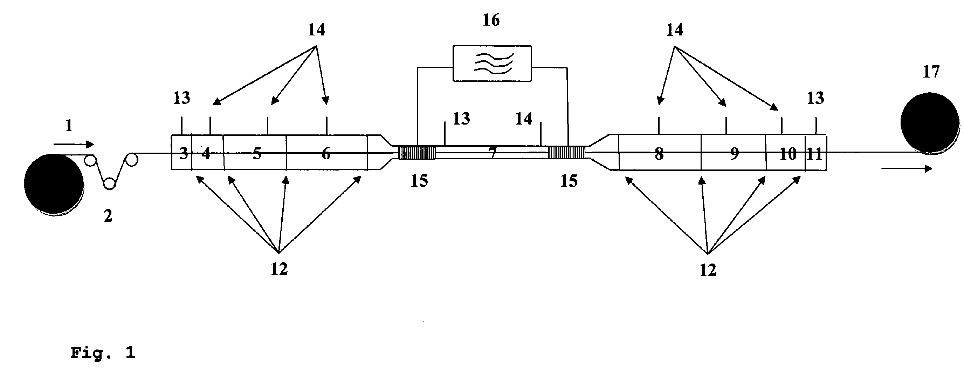

In one embodiment of the process, the vacuum system (shown in Fig. 1) contains seven consecutively interconnected compartments, and the pressure decreases from the first vacuum compartment **(4)** having a pressure of at most 300 mbar, e.g., 1-300 mbar, for instance 10-50 mbar,to a value of at most 1.0 mbar e.g., 0.01-1.0 mbar, for instance 0.05-0.5 mbar, or even less than 0.1 mbar in the second vacuum compartment **(5).** The vacuum compartment **(6)** preceding the central vacuum plasma ignition compartment **(7)** has a pressure of at most 0.5 mbar, e.g. 0.05-0.5 mbar, for instance 0.1-0.2 mbar. Likewise, pressure is set to a value of at most 1 mbar, e.g., 0.01-0.5 mbar, for instance 0.05-0.2 mbar, or even less than 0.1 mbar in the compartment **(8)** subsequent to the vacuum plasma ignition compartment, and increases to a value of at most 800 mbar, e.g., 0.5-800 mbar, for instance 10-300 mbar in the subsequent vacuum compartments **(9** and **10)** in the series. The vacuum compartments are evacuated by use of pumps.

In one embodiment of the process, the vacuum system contains seven consecutively interconnected cylindrical vacuum compartments **(4-10),** wherein the vacuum plasma ignition compartment **(7)** is the fourth compartment in said series. The length of the first and the seventh vacuum compartments **(4,10)** may be 20-300 mm, e.g., 150-200 mm, the length of the second and the sixth vacuum compartments **(5,9)** may be 100-800 mm, e.g., 365 mm;, the length of the third and the fifth vacuum compartments **(6,8)** may be 100-1,200 mm, e.g., 185 mm; and the length of the vacuum plasma ignition compartment **(7)** may be 10-500 mm, e.g. 20-200 mm, for instance 100 mm. The pressure is gradually decreased from the first to the third compartment, and gradually increased from the fifth to the seventh compartment.

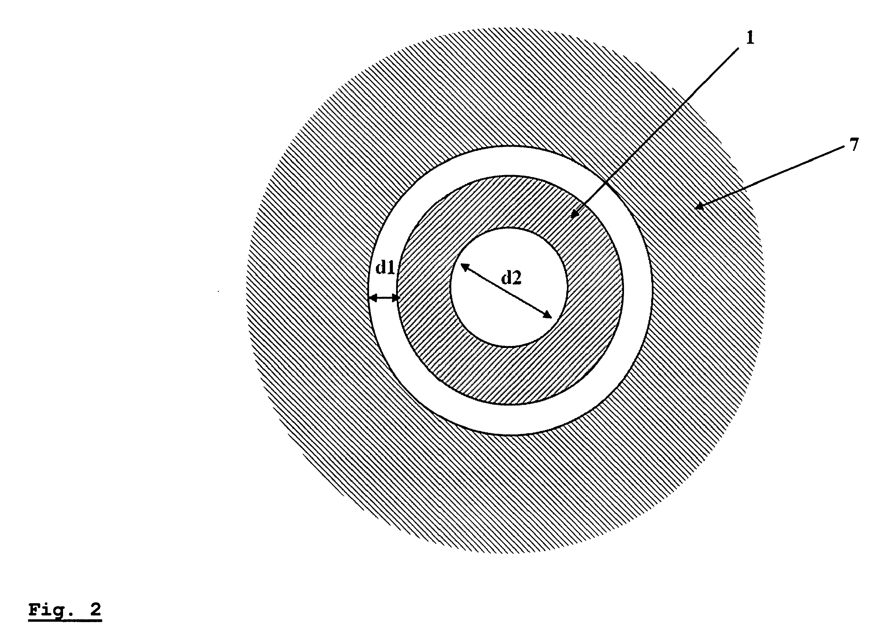

As shown in Fig. 2, the free cross-section dimension **d1** of the central compartment **(7),** the vacuum plasma ignition compartment, is smaller than the diameter **d2** of the lumen of the hollow fiber membrane **(1).** As a consequence of this geometry of the vacuum plasma ignition compartment **(7),** a plasma discharge is ignited only in the lumen. The pressure in the central compartment **(7)** filled with reactive gas is higher than the pressure in the adjacent compartments **(6,8),** i.e. the pressure within the plasma ignition zone is higher than the minimum pressure in the zone of reduced pressure having a negative pressure gradient towards the plasma discharge chamber **(7),** as seen from the entrance of the hollow fiber into the apparatus, and the zone of reduced pressure having a positive pressure gradient towards the exit of the hollow fiber from the apparatus. The gas pressure in the central compartment **(7),** in which the plasma is ignited, ranges from 0.005 to 1,200 mbar, e.g. 0.05 to 25 mbar, for instance 10 mbar, after the reactive gas has been introduced. The reactive gas flows into the lumen of the hollow fiber from the outside through the pores.

As a result of the inner diameter of the compartment **(7)** for plasma treatment of the hollow fiber lumen being only slightly larger than the outer diameter of the hollow fiber membrane, and the difference **d1** between the inner diameter of the compartment **(7)** and the outer diameter of the hollow fiber membrane being smaller than the inner diameter **d2** of the lumen of the hollow fiber, and the pressure in the compartment **(7)** being higher than in the adjacent compartments **(8,10),** the plasma is ignited inside the hollow fiber membrane and the reactive gas reacts with the membrane substrate to produce functional groups selectively and homogeneously bound to the lumen surface of the porous hollow fiber membrane.

Carrier gas or inert gas purge compartments **(3,11)** may be present preceding the first vacuum compartment **(4)** and subsequent to the last vacuum compartment **(10),** and a stream of a carrier gas, e.g. nitrogen, may be introduced into said compartments during the process. The carrier gas or inert gas purge compartments **(3,11)** may be connected to the vacuum compartments via aperture plates, feed ducts or pinhole diaphragms, the hollow fiber being passively conducted from one compartment to the next. Likewise, the individual vacuum compartments are separated from each other by aperture plates.

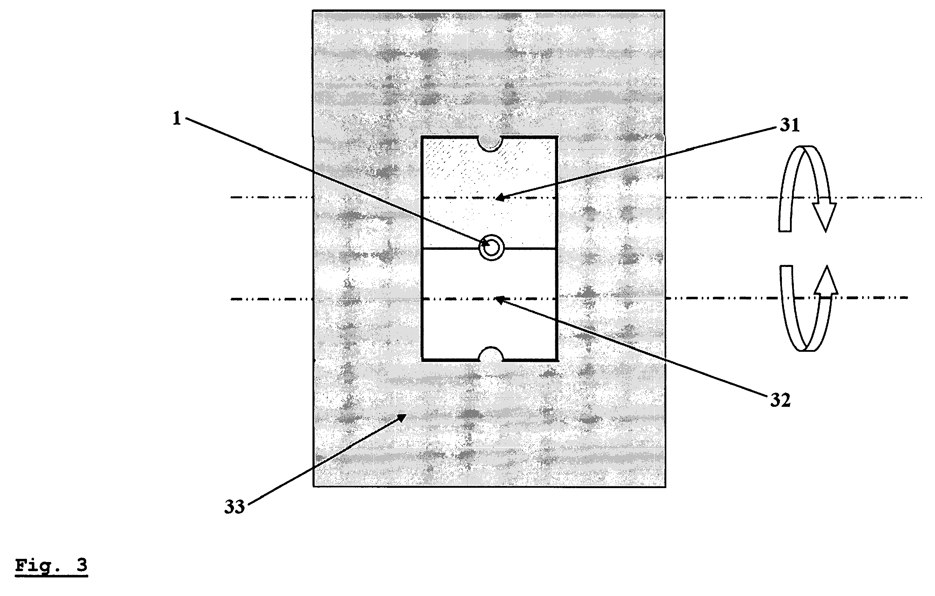

An alternative fiber transport mechanism is shown in Fig. 3. The fiber is conducted from one compartment to the next by moving rolls **(31,32)** which are in soft contact to each other and the hollow fiber (**1**). The rolls (**31,32**) are arranged within a vacuum-tight housing (**33**), which is flanged to the preceding and the subsequent vacuum compartment to provide a vacuum-tight seal.

The porous hollow fiber membrane is fed through the vacuum system at a velocity of from 0.1 to 100 m/min, e.g., 10 m/min. The residence time of the porous hollow fiber membrane in the plasma ignition zone may be 0.001 to 4 s, e.g., 0.04 s. For instance, if the plasma zone has a length of 6 mm and the hollow fiber is transported at a velocity of 0.5 m/min, the residence time of the fiber in the plasma is 0.7 s.

The hollow fibers may have an inner diameter of from 5 to 1,500 µm, e.g. from 70 to 700 µm, for instance, 200 to 400 µm; a wall thickness of 20 to 500 µm, e.g. 20 to 150 µm, for instance, 30 to 100 µm; an outer diameter of 100 to 2,000 µm, e.g. 110 to 1,000 µm, or 200 to 800 µm, for instance 260 µm; a pore diameter of 0.005 to 5.0 µm, e.g., 0.05 to 5.0 µm, for instance, 0.2 µm.

The hollow fiber membranes produced according to the present invention are made of a biocompatible polymeric material, e.g. polysulfone (PSU), polyethersulfone (PES), polyarylethersulfone (PAES), polyvinylpyrrolidone (PVP), polyurethane (PUR), polyamide (PA), polymethylmethacrylate (PMMA), polycarbonate (PC), polyacrylonitrile (PAN), polytetrafluoroethylene (PTFE), polypropylene (PP), polyvinyl acetate (PVA), cellulose acetate (CA), cellulose nitrate or regenerated cellulose. In one embodiment, hollow fibers comprising a mixture of polyarylethersulfone and polyvinylpyrrolidone are employed.

The length of the porous hollow fiber membrane substrate is not restricted and it may be continuously fed into the vacuum system in the apparatus according to the present invention directly from its production line. In the example below, the length of the membrane substrate is 1,000 m, but shorter or longer substrates can also be used.

The functional groups to be introduced on the lumen surface of the membrane substrate are preferably amino groups originating from reactive gases like ammonia, mixtures of hydrogen with nitrogen or ammonia, or amino compounds like diamines or triamines, e.g., diaminocyclohexane (DACH) and diethylenetriamine (DETA), but also from other organic precursors with primary amino groups, provided their vapor pressure is high enough to yield a substantial amount of the molecule containing the functional groups in the vapor phase. Further, functional groups other than amino groups can be introduced, e.g., carboxyl, hydroxyl, sulfonic acid, ester or epoxy groups, when reactive gases comprising corresponding functions are used instead of compounds containing amino functions.

Optionally, a stabilizing carrier gas may be mixed with the reactive gas before or in connection with the addition thereof into the plasma ignition compartment. Examples of suitable carrier gases are helium, nitrogen, hydrogen, and argon, or mixtures thereof.

The reactive gas or a mixture of the reactive gas and a carrier gas is introduced into the plasma ignition compartment (7) at a rate of from 0.1 to 100 sccm/min, e.g., 5 to 15 sccm/min.

The radio frequencies used for igniting and maintaining the plasma range from 1 kHz to 13.56 MHz or multiples thereof or 2.45 GHz. In one embodiment, a frequency of 375 kHz is used. The power output used is 0.1 to 50 W, e.g., 5-20 W, for instance 5-10 W.

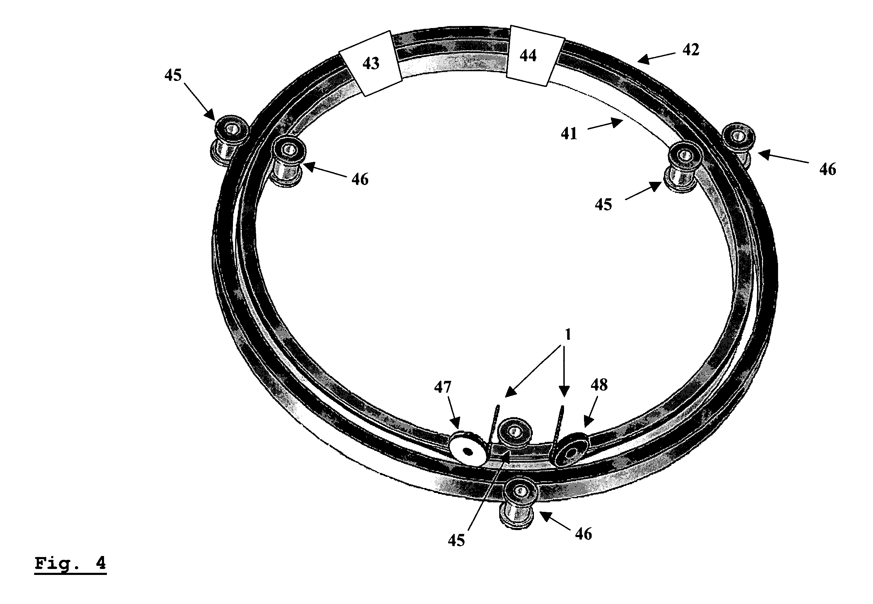

Alternatively, when the pressure in the plasma treatment chamber **(7)** is kept at about 1 bar, repetitive high voltage pulses can be applied using an electrode geometry similar to those shown in Figs. 1 and 4. The ratio between pulse-on time and pulse-off time has to be selected so that only a minimum of the electric energy input is transformed into heat but the energy of the free electrons formed is high enough to ionize and break chemical bonds.

The present invention also relates to an apparatus for carrying out the process of the present invention. The apparatus for the continuous plasma treatment of a hollow fiber membrane having an outer diameter OD comprises a plasma ignition compartment **(7)** comprising means **(13)** for introducing a gas into the compartment and means **(14)** for removing gas from said compartment, electrodes **(15)** for igniting and maintaining a plasma in said compartment being arranged outside said compartment. The apparatus further comprises a plurality of compartments **(4-10)** arranged in front of and behind the plasma ignition compartment **(7).** All compartments **(4-10)** of the apparatus are connected in series and each compartment has means **(14)** for removing gas from said compartment and is separated from the preceding and the subsequent compartment by aperture plates **(12).** A characteristic feature of the apparatus is that the diameter **d1** of the lumen of the hollow fiber **(1)** is larger than the free cross-section dimension **d2** of the volume between the hollow fiber **(1)** and the lumen of the plasma ignition compartment **(7).**

In one embodiment of the apparatus, the electrodes **(15)** used to generate the plasma are ring electrodes encircling the plasma ignition compartment **(7).** They generally have a distance from each other of 1 to 100 mm, e.g., 6 mm. Several electrodes of alternating polarity and arranged at variable spacing along the plasma ignition compartment can also be used. The voltage applied to the electrodes usually will be in the range of 50 to 5,000 V, e.g., 500 V.

In one embodiment of said apparatus shown in Fig. 1 it comprises seven cylindrical vacuum compartments **(4, 5, 6, 7, 8, 9,** and **10)** consecutively interconnected in series, wherein the vacuum plasma ignition compartment **(7)** is located centrally in said series.

Alternatively, the plasma ignition compartment can be formed by two concentric rotating rings **(41,42),** as shown in Fig. 4. The hollow fiber **(1)** is actively transported by the inner ring **(41)** moving at the same angular velocity as the outer ring **(42),** through the plasma ignition zone defined by electrodes **(43,44).** In this region both rings **(41,42)** are in close contact to each other and together form a gas-tight compartment. The rings are made from a non-conductive material and have a soft and elastic surface which actively encloses and transports the hollow fiber membrane **(1)** through the plasma ignition zone. The rings **(41,42)** are propelled by drive rollers **(45,46).** The hollow fiber membrane is fed to the assembly via a deflector roll **(47)** and the treated fiber leaves the assembly via another deflector roll **(48).** The whole assembly including the stock of hollow fiber membrane substrate and the treated fiber, which can be coiled on spools or reels, is preferably placed inside a closed compartment containing a controlled atmosphere, for instance, a glove box.

One embodiment of the present invention will now be disclosed in detail in connection with Fig. 1. A certain length of a porous hollow fiber membrane substrate **(1)** is fed into an apparatus comprising a vacuum system containing seven vacuum compartments **(4, 5, 6, 7, 8, 9,** and **10),** wherein the vacuum plasma ignition compartment **(7)** is centrally located in the line of compartments interconnected in series. In this embodiment, the other vacuum compartments are symmetrically arranged on both sides of the vacuum plasma ignition compartment **(7).** Said membrane substrate **(1)** is fed via a dancer roll **(2)** to a inert gas purge compartment **(3),** into which an inert gas, e.g., nitrogen is introduced in order to displace air introduced together with said membrane substrate **(1).** The membrane substrate **(1)** then passes through a first aperture plate **(12),** through a first vacuum compartment **(4),** a second aperture plate **(12),** a second vacuum compartment **(5),** a third aperture plate **(12),** a third vacuum compartment **(6)** and a fourth aperture plate **(12),** until it reaches the vacuum plasma ignition compartment **(7).**

The length of the first vacuum compartment **(4)** is 20-300 mm, e.g. 150-200 mm, such as about 185 mm, and it is connected via a conduit **(14)** to a vacuum line, e.g. leading to a rotary vane pump, creating a pressure in the vacuum compartment **(4)** of 5-300 mbar, e.g. 10-30 mbar, such as about 1-5 mbar. The above mentioned parameters for the vacuum compartment **(4)** also apply to the vacuum compartment **(10).**

The length of the vacuum compartment **5** is 100-750 mm, e.g. 365 mm, and it is connected via a conduit **(14)** to a vacuum line providing a pressure therein of 0.1-10 mbar, e.g. 0.4 mbar. The above mentioned parameters for the vacuum compartment **5** also apply to the vacuum compartment **9.**

The length of the vacuum compartment **6** is 50-1,200 mm, e.g. 185 mm, and it is connected via a conduit **(14)** to a vacuum line providing a pressure of 0.01-0.8 mbar, e.g. 0.1 mbar. The above mentioned parameters for the vacuum compartment **6** also apply to the vacuum compartment **8.**

The length of the vacuum plasma ignition compartment **7,** included in the series of vacuum compartments, is 1-2,000 mm, e.g. 20-1,000 mm, for instance 100 mm, or 800 to 1,500 mm. The vacuum plasma ignition compartment **7** also comprises two electrodes **(15)** connected to an RF generator **16.** The electrodes are metal foils circumventing a quartz tube representing the vacuum plasma ignition compartment **7.** In the vacuum plasma ignition compartment **7,** the reactive gas is introduced via conduit **13** to establish a pressure of 0.05-100 mbar, e.g. 0.5 to 25 mbar, for instance 10 mbar in the compartment **7.** Before the reactive gas enters the vacuum plasma ignition compartment **7,** the major part of the residual air present in this compartment, i.e. the ambient atmosphere which had leaked into compartment **7** from the adjacent vacuum compartments **6** and **8,** is displaced or evacuated.

The residence time of the porous hollow fiber membrane substrate **1** in the vacuum compartments **4** and **10** is 0.05-150 s, e.g. 0.1 to 10 s, for instance 1.1 s, in the vacuum compartments **5** and **9** 0.1-300 s, e.g. 0.5 to 10 s, for instance 2.2 s; in the vacuum compartments **6** and **8** 0.05-150 s, e.g. 0.1 to 10 s, for instance 1.1 s, and in the vacuum plasma ignition compartment **7** 0.5-650 s, e.g. 1 to 10 s, for instance 6 s.

In the vacuum plasma ignition compartment **7** the lumen surface of the porous hollow fiber membrane substrate **1** is functionalized in a homogeneous way with the functional groups, e.g. amino groups. Said surface is homogeneously provided with amino groups of a density of 0.5-10 µmol/g membrane, e.g. 4-7 µmol/g membrane, for instance 6.6 µmol/g membrane, as measured by polyelectrolyte titration. For the determination, the fibers are incubated with an excess of a solution of a negatively charged polyelectrolyte and subsequently titrated with a standard solution of a positively charged electrolyte to detect the inversion point.

After leaving the vacuum plasma ignition compartment **7** through a fifth aperture plate **(12),** the porous hollow fiber membrane produced is fed through vacuum compartment **8,** a sixth aperture plate **(12),** vacuum compartment **9,** a seventh aperture plate **(12),** vacuum compartment **10** and an eighth aperture plate **(12)** to a gas purge compartment **11,** to which an inert gas, e.g., nitrogen is added in order to prevent air from entering vacuum compartment **10.** The porous hollow fiber membrane produced is then fed to a fiber uptake assembly **17.**

In this way the plasma treatment process can be conducted continuously, and in principle as long as the porous hollow fiber substrate material is available at the entrance of the system. It is also possible to move the fiber back and forth several times. The total period for a membrane substrate **1** to pass the vacuum system is 1-1,600 s, e.g. 2-30 s, for instance 16 s per treatment cycle.

Thereafter, the porous hollow fiber membrane having a homogeneously functionalized lumen surface may be subjected to a further derivatization step, for instance a step in which ligands are bound to the functional groups. The ligands are coupled to the functional groups in a separate wet-chemical process, known to the person skilled in the art. In this way the lumen surface may selectively be furnished with a desired functionality, e.g., anti-coagulative properties. In one embodiment of the invention, a thrombin inhibitor, e.g., the synthetic thrombin inhibitor Argatroban, available under the trade name Argatra^{®} from Mitsubishi Pharma, is immobilized on the surface of the functionalized fiber and provides the fiber with anti-thrombogenic properties.

After functionalization, the fibers are usually arranged in modules e.g. containing a bundle of 10 up to more than 10,000 fibers. Experimental modules contain 10-100 fibers. Modules employed for blood treatment in a clinical setting contain more than 1,000 fibers. The porous hollow fiber membranes may be used to manufacture devices for therapeutic purposes like therapeutic apheresis, hemodialysis, or hemo(dia)filtration.

It is the merit of the present invention to provide a continuous process for selectively modifying the lumen surface of a porous hollow fiber membrane using a low-pressure plasma. Despite the relatively low RF power output used, the process allows for igniting and maintaining a plasma inside a hollow fiber having an inner diameter of less than 0.7 mm. This is achieved by a combination of the design of the reactor in which the plasma treatment takes place and the pressure distribution in the reactor. The plasma-forming gas is introduced into the lumen of the hollow fiber membrane from the outside through the pores of the hollow fiber.

It will be understood that the features mentioned above and those described hereinafter can be used not only in the combination specified but also in other combinations or on their own, without departing from the scope of the present invention.

The present invention will now be described in more detail in the examples below. It is to be understood that the examples are not intended to limit the scope of the present invention and are merely an illustration of a preferred embodiment of the invention.

### Examples

### Example 1

1000 m of a porous polyarylethersulfone-polyvinylpyrrolidone (PAES/PVP) hollow fiber membrane substrate **1** with an outer shell diameter of 260 µm and a wall thickness of 35 µm were fed through the apparatus shown in Fig. 1 at a velocity of 0.5 m/min. The vacuum plasma ignition compartment **7** had a length of 100 cm and a central segment of 12.5 cm had a diameter of 0.38 mm. Into said compartment **7** a mixture of ammonia and helium (18.2 vol% NH₃, 81.8 vol% He) was introduced at a rate of 11 sccm, generating a pressure of 12 mbar. The plasma was excited at a frequency of 375 kHz with a power of 5 W. The length of the plasma zone was 6 mm. The gas pressure in the different compartments of the apparatus is shown below:

| Compartment | **4** | **5** | **6** | **7** | **8** | **9** | **10** |
|---|---|---|---|---|---|---|---|
| Pressure [mbar] | 23 | 0.28 | 0.16 | 8 | 0.14 | 2.03 | 170 |

The modified hollow fiber membrane obtained was treated with 5% pentafluorobenzaldehyde (PFBA) in cyclohexane with a contact time of 2 hrs and Soxhlet extraction with cyclohexane over night. The element distribution on the lumen surface of the fiber before and after plasma treatment was measured using electron spectroscopy (ESCA). The results are shown below. Fluorine from PFBA reacts with amino groups, so a high fluorine content shows functionalization of the fiber.

| Element (%) | C | O | S | N | F |
|---|---|---|---|---|---|
| Untreated | 74.4 | 17.1 | 3.4 | 3.6 | 1.6 |
| He/NH₃-Plasma | 73.7 | 17.6 | 3.0 | 3.3 | 2.6 |

The functionalization of the membrane was determined by polyelectrolyte titration of the amino groups to be 6.6 µmol/g fiber. For the determination, the fibers were incubated with an excess of a solution of a negatively charged polyelectrolyte. Subsequently, titration with a positively charged electrolyte was performed to detect the inversion point, and functionalization density of the fibers was calculated.

The synthetic thrombin inhibitor Argatroban (Argatra® from Mitsubishi Pharma) was coupled to the amino functionalities on the lumen surface of the hollow fiber membrane via the carboxy group of the synthetic thrombin inhibitor. The protein coupling was performed in 25 mM MES buffer at pH 4.7 (MES = morpholino-ethane sulfonic acid). The synthetic thrombin inhibitor was dissolved in water in a concentration of 0.5 mg/ml. EDC and NHS as catalysts for the amid-binding were dissolved in a concentration of 2.5 mg/ml and 1 mg/ml, respectively. Per 1 mini module comprising 160 potted hollow fibers having a length of 17 cm each, 25 ml of coupling solution were used. The coupling was performed at room temperature for 3 hours under perfusion (flow rate 2.5 ml/min) in the membrane modules. After the coupling the membranes were flushed with 1 M sodium chloride solution and water (1 L of each). Finally, the modules were dried under vacuum at 40°C, sterilized and stored in the fridge. Immobilised Argatroban acc. to BCA-Assay: 2.6 µmol/g or 1.3 mg/g

The anti-thrombogenic effect of the functionalised fibers was determined using a chromogenic test. As active thrombin cleaves the substrate and catalyses formation of the chromophor p-nitroaniline, thrombin activity was measured by an increase in OD405 (optical density at 405 nm) in the sample. The membranes with immobilised synthetic thrombin inhibitor were incubated with an aqueous thrombin solution for 1 hour at 37°C. In comparison to non-functionalised fibers and the standard curve, a reduced thrombin activity is measured for the samples. Measured reduction of thrombin activity (in-vitro): 0.3 to 0.4 U per g fibers.

### Example 2

Example 1 was repeated using a porous polypropylene hollow fiber having an inner diameter of 280 µm and a wall thickness of 50 µm. Subsequent to the plasma treatment, the fiber was treated with 0.1 M H₂SO₄ for 0.5 h to oxidize any amino groups formed on the fiber surfaces. The element distribution on the outer surface and the lumen surface, respectively, of the fiber before and after treatment with sulfuric acid was measured using electron spectroscopy (ESCA). The results are shown below.

| Element (%) | C | C | C | C | C | N | N | O | S |
|---|---|---|---|---|---|---|---|---|---|
| eV | 288.7 | 288.3 | 287.1 | 285.9 | 284.6 | 400.8 | 399.8 | ges. | 168.9 |
| Outer surface | - | - | - | - | 100.0 | - | - | - | - |
| Lumen surface | - | 6.7 | 1.3 | 15.5 | 35.4 | - | 21.3 | 10.7 | - |
| Outer surface after H₂SO₄ | - | - | - | - | 97.7 | - | - | 2.3 | - |
| Lumen surface after H₂SO₄ | 5.0 | - | 7.8 | 9.7 | 28.7 | 10,3 | - | 32.0 | 6.5 |

The results show that amino groups have been formed on the lumen surface of the hollow fiber only. No amino groups have been formed on the outer surface of the hollow fiber.

## Claims

1. A continuous process for selectively modifying the lumen surface of a hollow fiber membrane **(1),** comprising the steps of
continuously transporting the hollow fiber membrane **(1)** through a zone **(4,5,6)** of reduced pressure having a negative pressure gradient and removing gas from the hollow fiber membrane;
continuously transporting the hollow fiber membrane **(1)** through a plasma ignition zone **(7)** comprising a reactive gas, optionally admixed with a carrier gas, and generating a plasma inside the lumen of the hollow fiber membrane **(1)** within said plasma ignition zone **(7),** thereby generating functional groups exclusively on the lumen surface of the hollow fiber membrane;
continuously transporting the hollow fiber membrane (1) through a zone **(8,9,10)** of reduced pressure having a positive pressure gradient.

2. The process of claim 1, wherein the inner diameter of the hollow fiber membrane is 5 to 1,500 µm.

3. The process of claim 1, wherein the hollow fiber membrane (1) comprises a polyarylethersulfone and a polyvinylpyrrolidone.

4. The process of claim 1, wherein the plasma is generated using radio frequencies at a power of 0.1 to 50 W.

5. The process of claim 1, wherein the pressure in the plasma ignition zone **(7)** is 0.05 to 100 mbar.

6. The process of claim 1, wherein the pressure within the plasma ignition zone **(7)** is higher than the minimum pressure in the zone **(4,5,6)** of reduced pressure having a negative pressure gradient and the zone **(8,9,10)** of reduced pressure having a positive pressure gradient, respectively.

7. The process of claim 1, wherein the reactive gas is ammonia and the inert gas is helium.

8. The process of claim 1, wherein the hollow fiber membrane **(1)** is transported at a velocity of 0.1 to 100 m/min.

9. An apparatus for selectively modifying the lumen surface of a hollow fiber membrane having an lumen diameter d2, comprising
a plasma ignition compartment **(7)** comprising
means **(13)** for introducing a gas into the compartment;
means **(14)** for removing gas from said compartment; and
electrodes **(15)** for igniting and maintaining a plasma in said compartment being arranged outside said compartment;
a plurality of compartments **(4,5,6,8,9,10)** arranged in front of and behind the plasma ignition compartment **(7) ;**
all compartments **(4,5,6,7,8,9,10)** of the apparatus being connected in series;
each compartment having means **(14)** for removing gas from said compartment; and
each compartment of the apparatus being separated from the preceding and the subsequent compartment by pinhole diaphragms **(12);**
**characterized in that** the free cross-section dimension d1 of the plasma ignition compartment **(7)** is smaller than the lumen diameter d2 of the hollow fiber membrane to be treated.

10. The apparatus of claim 9, further comprising inert gas purge compartments **(3,11)** comprising means **(13)** for introducing a gas into the inert gas purge compartment, said inert gas purge compartments **(3,11)** being arranged in front of the first compartment **(4)** and behind the last compartment **(10)** in the series, respectively.

11. The apparatus of claim 9 or 10, wherein the individual compartments **(3,4,5,6,8,9,10,11)** are separated by aperture plates **(12).**

12. A hollow fiber membrane obtainable by the process of any one of claims 1 to 8.

13. The hollow fiber membrane of claim 12, wherein the density of functional groups generated on the lumen surface is from 0.5 to 10 µmol/g membrane, as measured by polyelectrolyte titration.

14. The hollow fiber of claim 13, wherein the functional groups are amino groups.

15. Use of the hollow fiber membrane of any one of claims 12 to 14 in the manufacture of a device for therapeutic apheresis, hemodialysis, hemofiltration, or hemodiafiltration.
